(19) **Europäisches Patentamt**
European Patent Office
Office européen des brevets

(11) **EP 1 918 753 B1**

(12) **EUROPÄISCHE PATENTSCHRIFT**

(45) Veröffentlichungstag und Bekanntmachung des Hinweises auf die Patenterteilung:
**27.07.2011 Patentblatt 2011/30**

(51) Int Cl.:
**G02B 21/00** (2006.01)    **G01B 9/02** (2006.01)
**A61B 19/00** (2006.01)    **A61B 5/00** (2006.01)

(21) Anmeldenummer: **07019792.6**

(22) Anmeldetag: **10.10.2007**

(54) **Operationsmikroskop mit OCT-System**

Operation microscope with OCT system

Microscope d'opération doté d'un système OCT

(84) Benannte Vertragsstaaten:
**DE ES FR GB IT**

(30) Priorität: **06.11.2006 DE 102006052513**
**24.04.2007 DE 102007019678**

(43) Veröffentlichungstag der Anmeldung:
**07.05.2008 Patentblatt 2008/19**

(73) Patentinhaber: **Carl Zeiss Surgical GmbH**
**73447 Oberkochen (DE)**

(72) Erfinder:
• **Reimer, Peter**
**73479 Ellwangen (DE)**

• **Abele, Alfons**
**73527 Schwäbisch Gmünd (DE)**
• **Hauger, Christoph**
**73431 Aalen (DE)**
• **Seesselberg, Markus**
**73431 Aalen (DE)**

(74) Vertreter: **Gauss, Nikolai**
**c/o Carl Zeiss AG**
**Patentabteilung**
**Carl-Zeiss-Strasse 22**
**73447 Oberkochen (DE)**

(56) Entgegenhaltungen:
EP-A- 0 815 801     EP-A- 1 231 496
DE-A1-102004 049 368

**Beschreibung**

Die Erfindung betrifft ein Operationsmikroskop mit einem Beobachtungsstrahlengang und einem Mikroskop-Hauptobjektiv, das von dem Beobachtungsstrahlengang durchsetzt wird.

Ein Operationsmikroskop der eingangs genannten Art ist aus der DE 10 2004 049 368 A1 bekannt. Dort ist ein Operationsmikroskop mit einem Binokulartubus für Hauptbeobachtung und einem Binokulartubus für Mitbeobachtung beschrieben. Der Binokulartubus für Hauptbeobachtung und der Binokulartubus für Mitbeobachtung sind an einem gemeinsamen Operationsmikroskop-Grundkörper angeordnet. Der Binokulartubus für Hauptbeobachtung und der Binokulartubus für Mitbeobachtung haben stereoskopische Beobachtungsstrahlengänge. Diese durchsetzen ein gemeinsames Mikroskop-Hauptobjektiv.

In der EP 0 815 801 B1 ist ein Operationsmikroskop nach dem Oberbegriff von Anspruch 1 beschrieben, das ein OCT-System enthält.

Ein OCT-System erlaubt mittels optischer Kohärenztomographie die nichtinvasive Darstellung und Messung von Strukturen innerhalb eines Gewebes. Als optisches bildgebendes Verfahren ermöglicht die optische Kohärenztomographie insbesondere Schnitt- oder Volumenbilder von biologischem Gewebe mit Mikrometerauflösung zu erzeugen. Ein entsprechendes OCT-System umfasst eine Quelle für zeitlich inkohärentes und räumlich kohärentes Licht mit einer Kohärenzlänge $l_c$, die einem Probenstrahlengang und einem Referenzstrahlengang zugeführt wird. Der Probenstrahlengang ist auf das zu untersuchende Gewebe gerichtet. Laserstrahlung, die aufgrund von Streuzentren im Gewebe in den Probenstrahlengang zurückgestrahlt wird, überlagert das OCT-System mit Laserstrahlung aus dem Referenzstrahlengang. Durch die Überlagerung entsteht ein Interferenzsignal. Aus diesem Interferenzsignal lässt sich die Position von Streuzentren für die Laserstrahlung im untersuchten Gewebe bestimmen.

Für OCT-Systeme ist das Bauprinzip des "Time-Domain OCT" und des "Fourier-Domain OCT" bekannt.

Der Aufbau eines "Time-Domain OCT" ist beispielsweise in der US 5,321,501 anhand von Fig. 1a auf Sp. 5, Z. 40 - Sp. 11, Z. 10 beschrieben. In einem solchen System wird die optische Weglänge des Referenzstrahlenganges über einen schnell beweglichen Referenzspiegel fortlaufend variiert. Das Licht aus Proben- und Referenzstrahlengang wird auf einem Photodetektor überlagert. Wenn die optischen Weglängen von Proben- und Referenzstrahlengang übereinstimmen, entsteht auf dem Photodetektor ein Interferenzsignal.

Ein "Fourier-Domain OCT" ist beispielsweise in der WO 2006/10544 A1 erläutert. Um die optische Weglänge eines Probenstrahlenganges zu vermessen, wird wiederum Licht aus dem Probenstrahlengang Licht aus einem Referenzstrahlengang überlagert. Im Unterschied zu einem "Time-Domain OCT" wird jedoch für eine Messung der optischen Weglänge des Probenstrahlenganges das Licht aus Proben- und Referenzstrahlengang nicht direkt einem Detektor zugeführt, sondern zunächst mittels eines Spektrometers spektral zerlegt. Die so erzeugte spektrale Intensität des überlagerten Signals aus Proben- und Referenzstrahlengang wird dann mit einem Detektor erfasst. Durch Auswerten des Detektorsignals kann wiederum die optische Weglänge des Probenstrahlenganges ermittelt werden.

Das OCT-System des Operationsmikroskops nach der EP 0 815 801 B1 enthält eine Baugruppe zum Erzeugen eines OCT-Abtaststrahlengangs aus kurzkohärenter Laserstrahlung mit einer Analyseeinheit zur Auswertung von Interferenzsignalen. Dieser Baugruppe ist eine Einrichtung zum Scannen des OCT-Abtaststrahlengangs zugeordnet. Um mittels des OCT-Abtaststrahlengangs einen Operationsbereich abzutasten, enthält die Einrichtung zum Scannen zwei Scanspiegel, die um zwei Bewegungsachsen verstellt werden können. Bei dem Operationsmikroskop nach der EP 0 815 801 B1 wird der OCT-Abtaststrahlengang über einen Teilerspiegel in den Beleuchtungsstrahlengang des Operationsmikroskops eingekoppelt und mit diesem durch das Mikroskop-Hauptobjektiv hindurch zum Objektbereich gelenkt.

Aufgabe der Erfindung ist es, mit einem Operationsmikroskop das Erfassen von Tiefenbildern eines Objektbereichs zu ermöglichen.

Diese Aufgabe wird durch ein Operationsmikroskop nach Anspruch 1 gelöst.

Auf diese Weise ist es möglich, ein OCT-System in ein Operationsmikroskop zu integrieren, ohne dass in dem Operationsmikroskop optische Strahlengänge vignettiert werden und hierdurch Bildbeschnitt entsteht.

In Weiterbildung der Erfindung ist das Einkoppelelement als Planspiegel oder Teilerwürfel ausgebildet. Auf diese Weise wird ein Mitbeobachter stets eine freie Sicht zum Objektbereich ermöglicht.

In Weiterbildung der Erfindung umfasst das Operationsmikroskop einen Beobachtungsstrahlengang für Hauptbeobachtung und einen Beobachtungsstrahlengang für Mitbeobachtung, die das Mikroskop-Hauptobjektiv durchsetzen, wobei das Einkoppelelement in dem Beobachtungsstrahlengang für Mitbeobachtung angeordnet ist.

In Weiterbildung der Erfindung ist in dem Beobachtungsstrahlengang für Mitbeobachtung eine Optikbaugruppe angeordnet, um einen parallelen Beobachtungsstrahlengang in ein Zwischenbild zu überführen. Das Einkoppelelement in dem Beobachtungsstrahlengang für Mitbeobachtung ist dabei zwischen der Optikbaugruppe und dem Mikroskop-Hauptobjektiv angeordnet. Es kann aber auch zwischen der Optikbaugruppe und dem Zwischenbild vorgesehen sein.

In Weiterbildung der Erfindung umfasst das OCT-System für das Scannen des OCT-Abtaststrahlengangs einen ersten Scanspiegel. Vorzugsweise ist zusätzlich ein zweiter Scanspiegel vorgesehen, wobei der erste Scanspiegel um eine erste Drehachse bewegt werden kann und der zweite Scanspiegel sich um eine zweite Drehachse bewegen lässt. Dabei stehen die erste und die zweite Drehachse seitlich versetzt zueinander unter einem rechten Winkel. Auf diese Weise ist ein Abscannen eines Objektbereichs entsprechend einem senkrecht verlaufenden Rastermuster möglich.

In Weiterbildung der Erfindung umfasst das OCT-System einen Lichtleiter, der einen Lichtaustrittsabschnitt für den OCT-Abtaststrahlengang aufweist, wobei Mittel zum Bewegen des Lichtaustrittsabschnitts des Lichtleiters vorgesehen sind. Auf diese Weise lässt sich eine OCT-Abtastebene im Objektbereich variieren und es ist möglich, das System für unterschiedliche OCT-Wellenlängen in Anbetracht der für sichtbares Licht ausgelegten optischen Komponenten im Beobachtungsstrahlengang für Mitbeobachtung einzustellen.

In Weiterbildung der Erfindung ist in dem OCT-Abtaststrahlengang für das Einstellen einer geometrischen Abbildung eines Austrittsendes eines Lichtleiters in eine OCT-Abtastebene ein verstellbares optisches Element vorgesehen. Auf diese Weise kann die OCT-Abtastebene des Operationsmikroskop relativ zur Beobachtungsebene der optischen Beobachtungsstrahlengänge des Systems verlagert werden.

In Weiterbildung der Erfindung ist dem verstellbaren optischen Element eine Antriebseinheit zugeordnet. Auf diese Weise kann beispielsweise die OCT-Abtastebene um einen vorgegebenen Betrag relativ zur Beobachtungsebene des Operationsmikroskops variiert werden.

In Weiterbildung der Erfindung ist das OCT-System für das Bereitstellen eines ersten OCT-Abtastlichtstrahls mit einer ersten Wellenlänge und für das Bereitstellen eines zweiten OCT-Abtastlichtstrahles mit einer von der ersten Wellenlänge verschiedenen zweiten Wellenlänge ausgelegt. Auf diese Weise kann das Operationsmikroskop für die Untersuchung unterschiedlicher Gewebestrukturen und Körperorgane eines Patienten optimiert werden.

In Weiterbildung der Erfindung sind ein erstes und ein zweites OCT-System vorgesehen, die OCT-Abtastlichtstrahlen unterschiedlicher Wellenlänge bereitstellen. Auf diese Weise ist eine Untersuchung eines Objektbereichs auf der Grundlage unterschiedlicher OCT-Wellenlängen mit maximaler Auflösung möglich.

In Weiterbildung der Erfindung ist der OCT-Abtastlichtstrahl des ersten OCT-Systems einem rechten stereoskopischen Beobachtungsstrahlengang wenigstens teilweise überlagert und der OCT-Abtastlichtstrahl des zweiten OCT-System einem linken stereoskopischen Beobachtungsstrahlengang wenigstens teilweise überlagert, wobei von den Strahlengängen das Mikroskop-Hauptobjektiv in unterschiedlichen Bereichen durchsetzt wird. Vorzugsweise stellt dabei das erste OCT-System einen OCT-Abtastlichtstrahl der Wellenlänge $\lambda_1$ = 1300nm bereit und das zweite OCT-System einen OCT-Abtastlichtstrahl der Wellenlänge $\lambda_2$ = 800nm. Auf diese Weise kann mit dem Operationsmikroskop gleichzeitig der Schichtaufbau der Cornea und die Struktur der Retina an einem Patientenauge untersucht werden.

Vorteilhafte Ausführungsformen der Erfindung sind in den Figuren dargestellt und werden nachfolgend beschrieben.

Es zeigen:

Fig. 1    ein erstes Operationsmikroskop mit integriertem OCT-System;

Fig. 2    einen Schnitt des Mikroskop-Hauptobjektivs entlang der Linie II - II aus Fig. 1;

Fig. 3    einen Abschnitt des OCT-Systems in dem Operationsmikroskop;

Fig.4    eine Intensitätsverteilung des aus dem Lichtleiter des OCT-Systems im Operationsmikroskop austretenden OCT-Abtastlichtstrahls;

Fig. 5    eine Intensitätsverteilung des OCT-Abtastlichtstrahls in der OCT-Abtastebene im Objektbereich des Operationsmikroskops;

Fig. 6    ein zweites Operationsmikroskop mit integriertem OCT-System; und

Fig. 7    einen Abschnitt eines dritten Operationsmikroskops mit zwei integrierten OCT-Systemen.

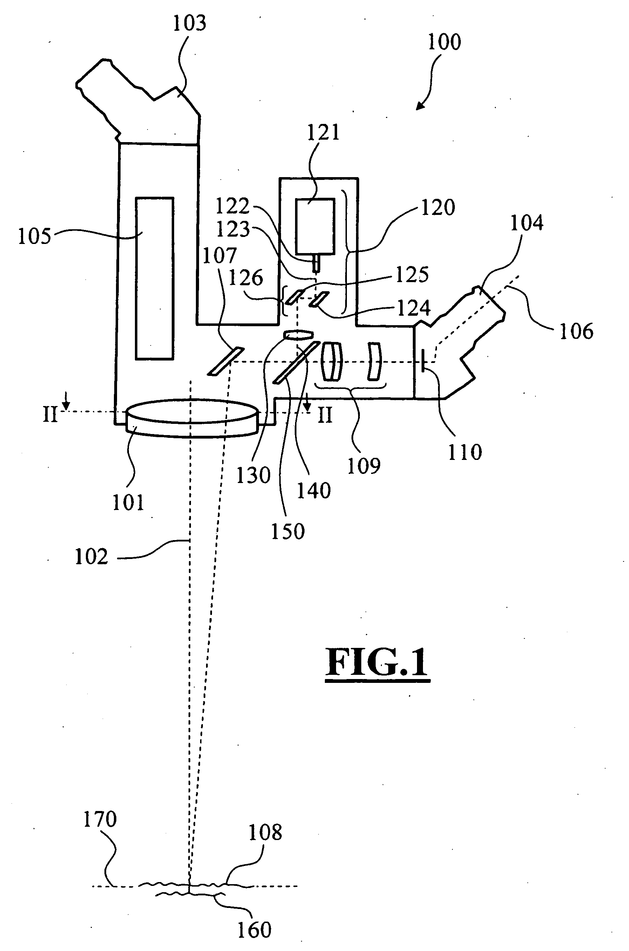

Das Operationsmikroskop 100 in Fig. 1 hat ein Mikroskop-Hauptobjektiv 101 mit einer optischen Achse 102. Das Mikroskop-Hauptobjektiv 101 hat eine Fokusebene 170 und wird von stereoskopischen Beobachtungsstrahlengängen eines Binokulartubus 103 für Hauptbeobachtung und eines Binokulartubus 104 für Mitbeobachtung durchsetzt. Dem Binokulartubus 103 für Hauptbeobachtung ist ein zoombares Vergrößerungssystem 105 zugeordnet. Die Fig. 1 zeigt den rechten Beobachtungsstrahlengang 106 des stereoskopischen Beobachtungsstrahlengangs vom Binokulartubus für Mitbeobachtung. Dieser Beobachtungsstrahlengang wird mittels eines auf der dem Objektbereich 108 abgewandten Seite des Mikroskop-Hauptobjektivs 101 angeordneten Umlenkspiegels 107 zum Objektbereich 108 gelenkt. In dem Beobachtungsstrahlengang 106 befindet sich ein Linsensystem 109. Das Linsensystem 109 bündelt den aus dem Objektbereich 108 durch das Mikroskop-Hauptobjektiv hindurch tretenden Beobachtungsstrahlengang 106, der nach dem Durchtreten des Mikroskop-Hauptobjektivs 101 parallel ist, zu einem Zwischenbild 110 im Binokulartubus 104 für Mitbeobachtung.

Das Operationsmikroskop 100 enthält ein OCT-System 120 zur Aufnahme von OCT-Bildern. Dieses OCT-System umfasst eine Einheit 121 für die Erzeugung und Analyse eines OCT-Abtaststrahlengangs. Die Einheit 121 ist in das Operationsmikroskop 100 integriert. Sie kann aber auch außerhalb des Operationsmikroskops, etwa in einer entsprechenden Stativkonsole angeordnet werden. Die Einheit 121 ist mit einem Lichtleiter 122 verbunden. Über diesen Lichtleiter 122 stellt die Einheit 121 einen OCT-Abtaststrahlengang bereit. Der aus dem Lichtleiter 122 austretende Abtaststrahlengang 123 wird auf einen ersten Scanspiegel 124 und einen zweiten Scanspiegel 125 einer OCT-Scaneinheit 126 geführt. Er durchtritt nach der OCT-Scaneinheit 126 eine Sammellinse 130. Die Sammellinse 130 bündelt den Abtaststrahlengang 123 zu einem parallelen Strahlenbündel 140.

Natürlich ist es auch möglich mit dem ersten Scanspiegel 124 und dem zweiten Scanspiegel 125 der OCT-Scaneinheit 126 einen parallelen OCT-Abtaststrahlengang abzulenken. Hierfür bedarf es einer geeigneten Sammellinse, die zwischen Lichtleiter 122 und OCT-Scaneinheit 126 angeordnet wird. Eine Sammellinse 130, die sich auf der dem Lichtleiter abgewandten Seite der OCT-Scaneinheit 126 befindet, ist dann nicht erforderlich. Das Strahlenbündel 140 aus der OCT-Scaneinheit 126 wird zu einem Teilerspiegel 150 geführt. Der Teilerspiegel 150 ist im Beobachtungsstrahlengang 106 angeordnet. Der Teilerspiegel 150 ist im wesentlichen durchlässig für den für den Menschen sichtbaren Spektralbereich von Beobachtungslicht in diesem Beobachtungsstrahlengang. Er reflektiert jedoch den OCT-Abtaststrahlengang und überlagert diesen dem Beobachtungsstrahlengang 106. Der Teilerspiegel 150 kann als Spiegelelement mit Planplatten aber auch als Teilerwürfel ausgeführt sein.

Das Licht des OCT-Abtaststrahlengangs 123 wird durch das Mikroskop-Hauptobjektiv 101 in einer OCT-Abtastebene 160 gebündelt. Die OCT-Abtastebene 160 ist die Ebene der durch die optischen Elemente im OCT-Abtaststrahlengang mit OCT-Scaneinheit 126, Sammellinse 130, Teilerspiegel 150, Umlenkspiegel 107 und Mikroskop-Hauptobjektiv 101 festgelegten geometrischen Abbildung des Austrittsendes von Lichtleiter 123 in den Objektbereich. Das heißt, das entsprechende geometrische Bild des Lichtleiter-Austrittsendes liegt in der OCT-Abtastebene 160.

Das in den OCT-Abtaststrahlengang zurückgestreute Licht gelangt über den Umlenkspiegel 107 und den Teilerspiegel 150 zurück in die Einheit 121. Dort wird das aus dem Objektbereich zurückgestreute OCT-Abtastlicht mit OCT-Strahlung aus einem Referenzstrahlengang interferiert. Das Interferenzsignal wird mittels eines Detektors erfasst und durch eine Rechnereinheit ausgewertet, die aus diesem Signal eine optische Weglängedifferenz zwischen Streuzentren für OCT-Licht im Objektbereich und der Weglänge von Licht im Referenzzweig bestimmt.

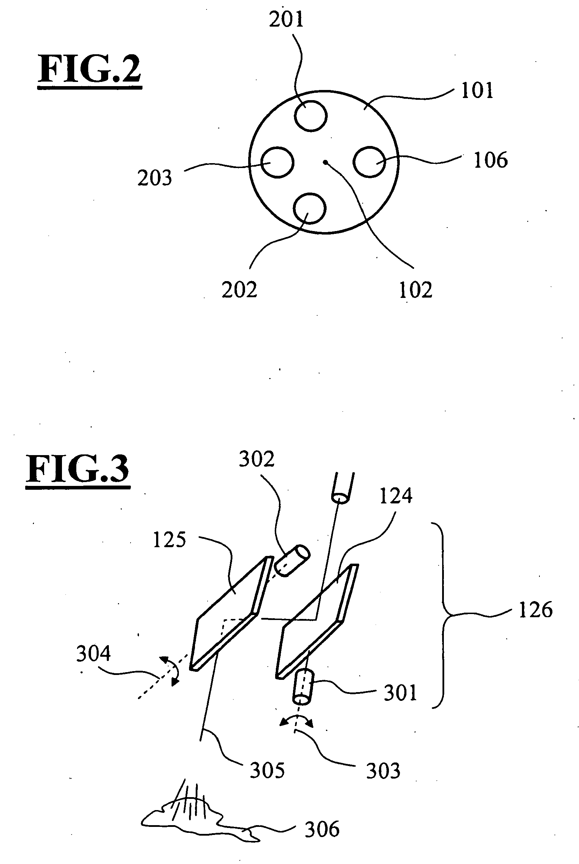

Fig. 2 ist ein Schnitt entlang der Linie II - II aus Fig. 1. Diese Figur erläutert den Verlauf der stereoskopischen Beobachtungsstrahlengänge aus Binokulartubus 103 und Binokulartubus 104 des Operationsmikroskops 100 auf Fig. 1. Die optische Achse 102 des Mikroskop-Hauptobjektivs 101 liegt in dessen Zentrum. Der stereoskopische Strahlengang für Hauptbeobachtung 201, 202 und der stereoskopische Strahlengang für Mitbeobachtung 203, 106 durchsetzen das Mikroskop-Hauptobjektiv 101 in voneinander getrennten Schnittbereichen.

Fig. 3 zeigt die Scanspiegeleinheit 126 des Operationsmikroskops 100 auf Fig. 1. Der erste Scanspiegel 124 und der zweite Scanspiegel 125 sind mittels Stellantrieben 301, 302 um zwei zueinander senkrecht verlaufende Achsen 303, 304 drehbeweglich angeordnet. Dies ermöglicht, den OCT-Abtaststrahlengang 305 über eine Ebene 306 zu scannen.

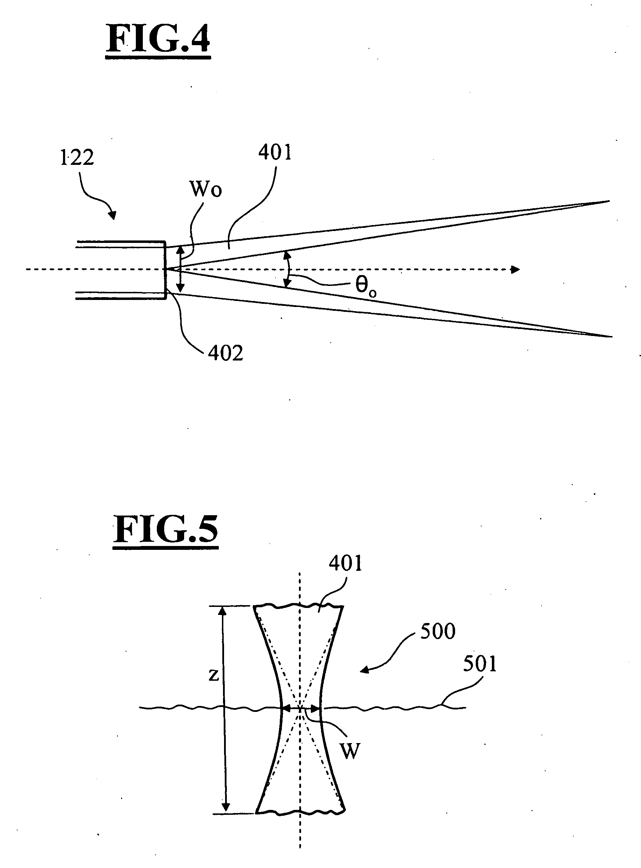

Fig. 4 zeigt den Frontabschnitt 402 des Lichtleiters 122 aus Fig. 1. Der Lichtleiter 122 wirkt für Licht der Wellenlänge $\lambda$ = 1310nm als Monomodenfaser. Der Durchmesser d des Faserkerns des Lichtleiters 122 genügt der Beziehung

$$\frac{d}{2} < 2.4 \frac{\lambda}{2\pi NA},$$

Wobei NA die numerische Apertur der Frontfläche des Lichtleiters ist. Vorzugsweise liegt der Durchmesser d des Faserkerns des Lichtleiters 122 im Bereich $5\mu m < d < 10\mu m$. In diesem Parameterbereich führt der Lichtleiter 122 das Licht mit gaussförmigen Wellenmoden. Aus dem Lichtleiter 122 tritt der OCT-Abtastlichtstrahl 401 mit einem näherungsweise gaussförmigen Strahlungsprofil aus, das durch einen Taillenparamter $W_0$ und einem Öffnungsparameter $\theta_0$ charakterisiert ist, wobei gilt

$$\theta_0 = \frac{2\lambda}{\pi W_0}$$

Für einen Faserkerndurchmesser von $d_0$ = 10$\mu$m und einer Wellenlänge $\lambda_0$ = 1310nm ergibt sich damit als Maß für die Strahldivergenz ein Öffnungswinkel von $\theta_0 \approx$ 0,0827 rad.

Die Frontfläche 402 des Lichtleiters 122 wird über die Scanspiegel 124 und 125 im Operationsmikroskop 100 aus Fig. 1, die Sammellinse 130, den Teilerspiegel 150, den Umlenkspiegel 107 und das Mikroskop-Hauptobjektiv 101 auf den Objektbereich 108 in die OCT-Abtastebene 160 abgebildet.

Die Fig. 5 zeigt den Verlauf der Intensitätsverteilung des OCT-Abtastlichtstrahls 401 senkrecht zur OCT-Abtastebene 501. In der OCT-Abtastebene 501 hat die Intensitätsverteilung der OCT-Abtaststrahlung eine kleinste Einschnürung. Außerhalb der OCT-Abtastebene nimmt der Durchmesser des OCT-Abtaststrahlengangs zu. Da der OCT-Abtastlichtstrahl 401 aus dem Lichtleiter 122 aus Fig. 4 mit einem näherungsweise gaussförmigen Strahlungsprofil austritt, bewirken die Sammellinse 130 und das Mikroskop-Hauptobjektiv 101 für den OCT-Abtastlichtstrahl im Bereich der OCT-Abtastebene 160 ein sogenanntes Gaussbündel 500 des OCT-Abtastlichtstrahls 401. Dieses Gaussbündel 500 ist durch den konfokalen Parameter z als Maß für die longitudinale Ausdehnung der Taille des Gaussbündels, sowie den Taillenparameter W als Mass für den Durchmesser der kleinsten Einschnürung 502 des OCT-Abtastlichtstrahls 401, d.h. für den Durchmesser von dessen Taille, charakterisiert, wobei gilt:

$$z = 2\frac{W^2\pi}{\lambda},$$

dabei ist λ die Wellenlänge des OCT-Abtastlichtstrahls. Zwischen dem Taillenparametern W des Gaussbündels 500 und dem Taillenparameter Wo des in Fig. 4 gezeigten, aus dem Lichtleiter 122 austretenden Abtastlichtstrahls 401 gilt die folgende Beziehung:

$$W = \beta W_0,$$

wobei β der Vergrößerungs- bzw. Verkleinerungsparameter der oben erwähnten geometrischen Abbildung des Austrittsendes von Lichtleiter 122 aus Fig. 1 in der OCT-Abtastebene ist. β ist mit der Brennweite $f_1$ der Sammellinse 130 aus Fig. 1 und der Brennweite $f_2$ des Mikroskop-Hauptobjektivs über die folgende Beziehung verknüpft:

$$\frac{f_2}{f_1} = \beta$$

Die Größe von Strukturen, die sich mit dem OCT-Abtastlichtstrahl 401 auflösen lassen, ist durch dessen Durchmesser in der OCT-Abtastebene 160, d.h. durch den Taillenparameter W bestimmt. Erfordert beispielsweise eine Applikation eine Lateralauflösung des OCT-Systems im Operationsmikroskop von ca. 40μm, muss nach dem Nyquist-Theoren der Querschnitt des OCT-Abtastlichtstrahls 401 auf der Oberfläche ca. 20μm betragen. Bei gegebener Wellenlänge λ für den OCT-Abtastlichtstrahl 123 aus Fig. 1 müssen daher für eine gewünschte Auflösung des OCT-Systems 120 die Vergrößerung der optischen Abbildung im OCT-Strahlengang und der Durchmesser des Faserkerns im Lichtleiter 122 geeignet gewählt werden.

Der konfokale Parameter z als Maß für die longitudinale Ausdrehung der Taille des Gaussbündels bestimmt den axialen Tiefenbereich, aus dem in dem OCT-Abtaststrahlengang 123 aus Fig. 1 zurückgestreutes Licht detektiert werden kann: Je kleiner der konfokale Parameter z, desto größer ist der Verlust des OCT-Systems an lateraler Auflösung bei Entfernung eines mit OCT-Abtaststrahlung abgetasteten Objekts von der OCT-Abtastebene 160, da sich der Ort von Streuzentren nur innerhalb des durch den Taillenparamter W und den konfokalen Parameter z definierten "Trichter" lokalisieren lässt.

Nachdem die axiale Auflösung eines OCT-Systems einerseits durch die Kohärenzlänge $l_c$ des Lichts der im OCT-system eingesetzten Lichtquelle begrenzt ist, andererseits die laterale Auflösung des OCT-Systems abnimmt, wenn dessen Tiefenhub die mit dem konfokalen Parameter z gegebene Ausdehnung übersteigt, ist das Einstellen des konfokalen Parameters z auf den Tiefenhub des OCT-Systems günstig.

Für eine bestimmte Wellenlänge λ des OCT-Abtastlichtstrahls 401 ergibt sich dann die mögliche laterale Auflösung des OCT-Systems aus Fig. 1, da die Wellenlänge λ und der konfokale Parameter z den Taillenparameter W festlegen. Die Optikeinheiten im OCT-Abtaststrahlengang 123 aus Fig. 1 und die Bemassung des Faserkerns von Lichtleiter 122 sind dann so zu wählen, dass sich der betreffende Taillenparameter W ergibt.

Das Operationsmikroskop 100 ist so ausgelegt, dass die Fokusebene 170 des Mikroskop-Hauptobjektivs 101 für den sichtbaren Spektralbereich und OCT-Abtastebene 160 zusammenfällt. Dann liegt die in Fig. 5 gezeigte Taille 502 des OCT-Abtastlichtstrahls in der Fokusebene des Operationsmikroskops.

Alternativ zu dieser Auslegung des Operationsmikroskops kann auch ein Versatz von OCT-Abtastebene und Fokusebene des Operationsmikroskops vorgesehen sein. Vorzugsweise ist dieser Versatz nicht größer als der konfokale Parameter z des OCT-Abtastlichtstrahls im Bereich der OCT-Abtastebene. Dies ermöglicht es beispielsweise einen unmittelbar unter der Fokusebene des Operationsmikroskops liegenden Objektbereich mittels OCT zu visualisieren. Es kann aber auch sinnvoll sein, für bestimmte Anwendungen einen definierten Versatz vorzusehen, der den konfokalen Parameter übersteigt, etwa um mit dem Operationsmikroskop die Vorderseite der Cornea eines Patientenauges untersuchen zu können und gleichzeitig mittels des OCT-Systems die Rückseite der Cornea des Patientenauges oder dessen Linse zu visualisieren.

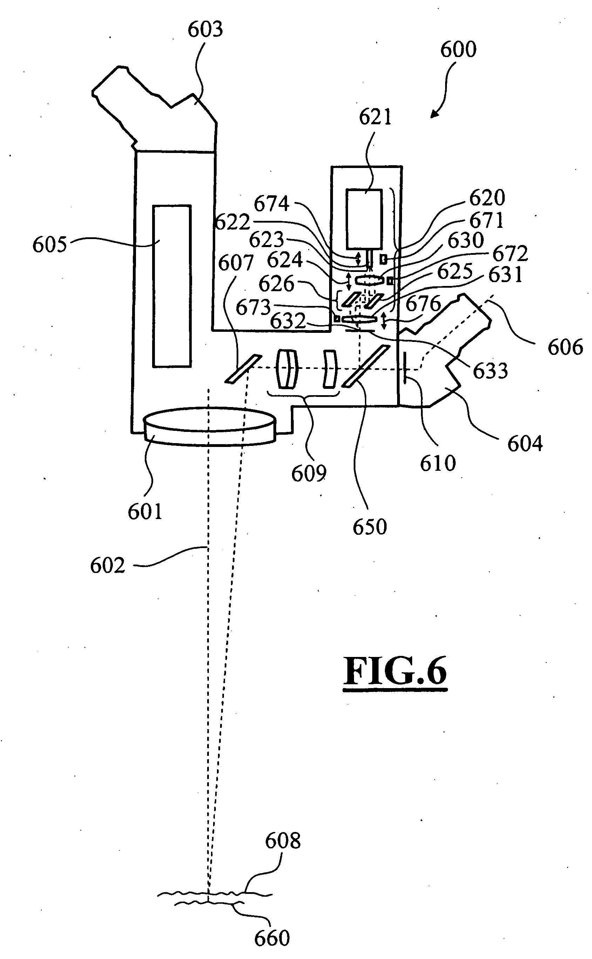

Die Fig. 6 zeigt ein weiteres Operationsmikroskop 600 mit integrierte OCT-System 620. Soweit Baugruppen des Operationsmikroskop 600 mit Baugruppen im Operationsmikroskop 100 aus Fig. 1 übereinstimmen, sind die Baugruppen dort mit im Vergleich zu Fig. 1 um die Zahl 500 erhöhten Zahlen als Bezugszeichen versehen.

Anders als beim Operationsmikroskop 100 aus Fig. 1 ist beim Operationsmikroskop 600 ein Teilerspiegel 650 vorgesehen, der sich im konvergenten Beobachtungsstrahlengang zwischen einem Tubuslinsensystem 609 und dem von diesem erzeugten Zwischenbild 610 des Objektbereichs befindet. Das OCT-System 620 umfasst eine Einheit 621 für die Erzeugung eines OCT-Abtaststrahlengangs 623 in zwei unterschiedlichen Wellenlängenbereichen: Es vermag einen OCT-Abtastlichtstrahl mit Wellenlänge $\lambda = 800nm$ und einen OCT-Abtastlichtstrahl mit Wellenlänge $\lambda = 1310nm$ zu erzeugen.

Der aus dem Lichtleiter 622 austretende OCT-Abtaststrahlengang 623 wird über ein erstes OCT-Linsensystem 630 auf die Scanspiegeleinheit 626 mit Scanspiegeln 624, 625 gelenkt. Aus der Scanspiegeleinheit 626 gelangt er zu einem zweiten OCT-Linsensystem 631. Die OCT-Linsensysteme 630, 631 bewirken eine Zwischenabbildung 632 des Lichtleiter-Austrittsendes in einer Ebene 633, die zu der OCT-Abtastebene 660 im Objektbereich 608 konjugiert ist.

Um einer Bedienperson die Einstellung der OCT-Abtastebene 660 im Bezug auf die Objektebene 608 der optischen Beobachtungsstrahlengänge im Operationsmikroskop 600 zu ermöglichen, ist eine Verstellbarkeit der Linsensysteme 630, 631 und des Austrittsendes von Lichtleiter 622 vorgesehen. Hierzu umfasst das Operationsmikroskop 600 Antriebseinheiten 671, 672, 673, die den Linsensystemen 630, 631 und dem Lichtleiter 622 zugeordnet sind. Mittels dieser Antriebseinheiten 671, 672, 673 können die Linsensysteme 630, 631 und der Lichtleiter 622 entsprechend der Doppelpfeile 674, 675, 676 verlagert werden. Insbesondere kann hierdurch nicht nur die Lage der OCT-Abtastebene 660 variiert werden, sondern es lässt sich auch eine Vergrößerung bzw. Verkleinerung des Austrittsendes von Lichtleiter 622 auf einen gewünschten Wert einstellen.

Eine modifizierte Ausführungsform des anhand von Fig. 6 erläuterten Operationsmikroskops 600 enthält ein fokussierbares Mikroskop-Hauptobjektiv mit einstellbarer Brennweite. Auch diese Maßnahme ermöglicht das Verlagern einer OCT-Abtastebene und das Verändern der geometrischen Abbildung des Lichtleiter-Austrittsendes in die OCT-Abtastebene.

Bei einem Verlagern der OCT-Abtastebene des OCT-Systems 620 in dem Operationsmikroskop 600 wird vorzugsweise der vorliegend nicht gezeigte Referenzstrahlengang des Systems nachjustiert, damit dieser stets auf die eingestellte OCT-Abtastebene angepasst ist.

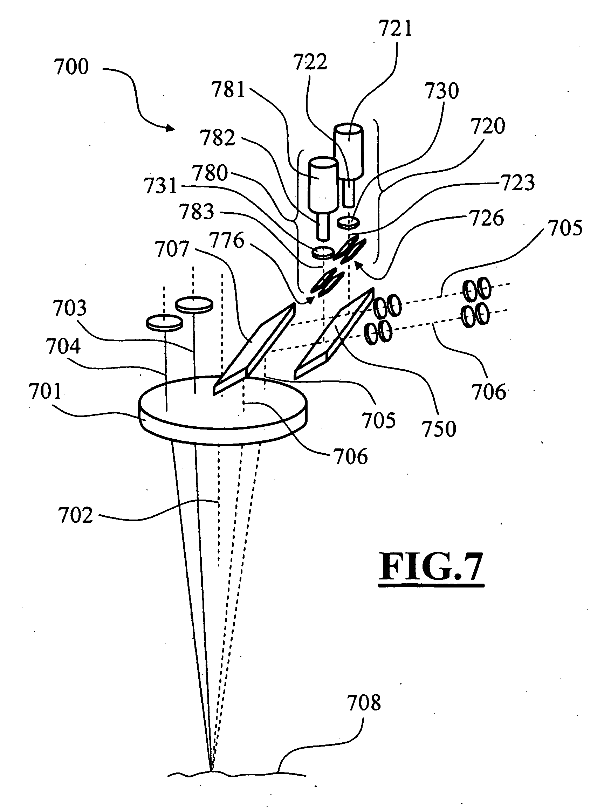

Die Fig. 7 zeigt einen Abschnitt 700 eines dritten Operationsmikroskops, in dem zwei OCT-Systeme 720, 780 vorgesehen sind. Das Operationsmikroskop hat wiederum ein Mikroskop-Hauptobjektiv 701 mit einer optischen Achse 702. Das Objektiv 701 wird von einem linken und rechten stereoskopischen Beobachtungsstrahlengang für Hauptbeobachtung 703, 704 und einem linken und rechten stereoskopischen Beobachtungsstrahlengang für Mitbeobachtung 705, 706 durchsetzt. Der stereoskopische Beobachtungsstrahlengang für Mitbeobachtung 705, 706 wird mittels eines auf der dem Objektbereich 708 abgewandten Seite des Mikroskop-Hauptobjektivs 701 angeordneten Umlenkspiegel 707 zum Objektbereich 708 gelenkt.

Die OCT-Systeme 720, 780 umfassen jeweils eine Einheit 721, 781 für die Erzeugung und Analyse eines OCT-Abtaststrahlengangs. Diese Einheiten 721, 781 stellen über Lichtleiter 722, 782 einen ersten OCT-Abtaststrahl 723 und einen zweiten OCT-Abtaststrahl 783 mit unterschiedlichen Wellenlängen $\lambda_1, \lambda_2$ bereit. Die OCT-Abtaststrahlen 723, 783 werden über Sammellinsen 730, 731 und OCT-Scaneinheit 726, 776 mit Scanspiegeln auf einen Teilerspiegel 750 gelenkt.

Der Teilerspiegel 750 ist im stereoskopischen Beobachtungsstrahlengang für Mitbeobachtung 705, 706 angeordnet. Er ist im wesentlichen durchlässig für den für den Menschen sichtbaren Spektralbereich von Beobachtungslicht, reflektiert jedoch die OCT-Abtaststrahlen 723, 783 derart, dass diese den Beobachtungsstrahlengängen 705, 706 überlagert werden und mit diesen das Mikroskop-Hauptobjektiv 701 durchsetzen.

Das in die OCT-Abtaststrahlengänge 723, 783 aus dem Objektbereich 708 zurückgestrahlte Licht wird in den Einheiten 721, 781 für die Erzeugung und Analyse des betreffenden OCT-Abtaststrahlenganges ausgewertet.

Der Einsatz von zwei OCT-Systemen ermöglicht, einen Objektbereich mit OCT-Licht unterschiedlicher Wellenlänge abzutasten, wobei für einen jeden OCT-Abtaststrahlengang eine für maximale Auflösung optimale Wahl von Lichtwellenlänge $\lambda_1, \lambda_2$ und der konfokale Parameter $z_1, z_2$ und der Taillenparamter $W_1, W_2$ getroffen werden kann.

## Patentansprüche

1. Operationsmikroskop (100, 600, 700) mit

   - einem Beobachtungsstrahlengang (106, 203, 606, 705, 706); und
   - einem Mikroskop-Hauptobjektiv (101, 601, 701), das von dem Beobachtungsstrahlengang (106, 203, 606, 705, 706) durchsetzt wird; und
   - einem OCT-System (120, 620, 720, 780) zur Untersuchung eines Objektbereichs (108, 608, 708), wobei das OCT-System (120, 620, 720, 780) einen OCT-Abtaststrahlengang (123, 623, 723, 783) aufweist, der durch das Mikroskop-Hauptobjektiv (101, 601) geführt ist,

   **dadurch gekennzeichnet, dass**

   - in dem Beobachtungsstrahlengang (106) ein Einkoppelelement (150, 650, 750) vorgesehen ist, um den OCT-Abtaststrahlengang (123, 623, 723, 783) in den Beobachtungsstrahlengang (106, 606, 705, 706) einzukoppeln und durch das

- Mikroskop-Hauptobjektiv (101, 601, 701) zum Objektbereich (108, 608, 708) zu führen,
- das Einkoppelelement als Teilerspiegel ausgebildet ist, der im Beobachtungsstrahlengang (106) angeordnet ist und der im Wesentlichen durchlässig ist für den für den Menschen sichtbaren Spektralbereich von Beobachtungslicht,
- der Teilerspiegel den OCT-Abtaststrahlengang reflektiert und diesen dem Beobachtungsstrahlengang (106, 606, 705, 706) überlagert.

2. Operationsmikroskop nach Anspruch 1, **dadurch gekennzeichnet, dass** das Einkoppelelement als Planspiegel oder Teilerwürfel ausgebildet ist.

3. Operationsmikroskop nach Anspruch 1 oder 2, **dadurch gekennzeichnet, dass** ein Beobachtungsstrahlengang (201, 202, 703, 704) für Hauptbeobachtung und ein Beobachtungsstrahlengang (106, 203, 606, 705, 706) für Mitbeobachtung vorgesehen sind, die beide das Mikroskop-Hauptobjektiv (101, 601, 701) durchsetzen, wobei das Einkoppelelement (150, 650, 750) in dem Beobachtungsstrahlengang (106, 203, 606, 705, 706) für Mitbeobachtung angeordnet ist.

4. Operationsmikroskop nach Anspruch 3, **dadurch gekennzeichnet, dass** in dem Beobachtungsstrahlengang (106, 606) für Mitbeobachtung eine Optikbaugruppe (109, 609) angeordnet ist, um einen parallelen Beobachtungsstrahlengang in ein Zwischenbild (110, 610) zu überführen,

5. Operationsmikroskop nach Anspruch 4, **dadurch gekennzeichnet, dass** das Einkoppelelement (150) in dem Beobachtungsstrahlengang (106) für Mitbeobachtung zwischen der Optikbaugruppe (109) und dem Mikroskop-Hauptobjektiv (101) angeordnet ist.

6. Operationsmikroskop nach Anspruch 4, **dadurch gekennzeichnet, dass** das Einkoppelelement (650) in den Beobachtungsstrahlengang (606) für Mitbeobachtung zwischen der Optikbaugruppe (609) und dem Zwischenbild (610) angeordnet ist.

7. Operationsmikroskop nach einem der Ansprüche 1 bis 6, **dadurch gekennzeichnet, dass** das OCT-System (120, 620) für das Scannen des OCT-Abtaststrahlenganges einen ersten Scanspiegel (124, 624) umfasst, der um wenigstens eine erste Drehachse (303) bewegt werden kann.

8. Operationsmikroskop nach Anspruch 7, **dadurch gekennzeichnet, dass** ein zweiter Scanspiegel (124, 125, 624, 625) vorgesehen ist, der um eine zweite Drehachse (304) bewegt werden kann, wobei die erste Drehachse (303) und die zweite Drehachse (304) seitlich versetzt in einem rechten Winkel zueinander stehen.

9. Operationsmikroskop nach einem der Ansprüche 1 bis 8, **dadurch gekennzeichnet, dass** das OCT-System (120, 620) einen Lichtleiter (122, 622) umfasst, der einen Lichtaustrittsabschnitt für den OCT-Abtaststrahlengang aufweist, wobei Mittel zum Bewegen des Lichtaustrittsabschnitts des Lichtleiters vorgesehen sind.

10. Operationsmikroskop nach einem der Ansprüche 1 bis 9, **dadurch gekennzeichnet, dass** in dem OCT-Abtaststrahlengang (623) für das Einstellen einer geometrischen Abbildung eines Austrittsendes eines Lichtleiters (622) in eine OCT-Abtastebene (660) ein verstellbares optisches Element (630, 631) vorgesehen ist.

11. Operationsmikroskop nach Anspruch 10, **dadurch gekennzeichnet, dass** dem verstellbaren optischen Element (630, 631) eine Antriebseinheit (672, 673) zugeordnet ist.

12. Operationsmikroskop nach einem der Ansprüche 1 bis 11, **dadurch gekennzeichnet, dass** das OCT-System (120, 620) für das Bereitstellen eines ersten OCT-Abtastlichtstrahls (623) mit einer ersten Wellenlänge und für das Bereitstellen eines zweiten OCT-Abtastlichtstrahles (623) mit einer von der ersten Wellenlänge verschiedenen zweiten Wellenlänge ausgelegt ist.

13. Operationsmikroskop nach einem der Ansprüche 1 bis 12, **dadurch gekennzeichnet, dass** ein erstes OCT-System (720) und ein zweites OCT-System (780) vorgesehen sind, die OCT-Abtastlichtstrahlen (723, 783) unterschiedlicher Wellenlänge bereitstellen.

14. Operationsmikroskop nach Anspruch 13, **dadurch gekennzeichnet, dass** der OCT-Abtastlichtstrahl (723) des ersten OCT-Systems (720) einem rechten stereoskopischen Beobachtungsstrahlengang (705) wenigstens teilweise überlagert ist und mit diesem das Mikroskop-Hauptobjektiv (701) des Operationsmikroskops (700) durchsetzt und der OCT-Abtastlichtstrahl (783) des zweiten OCT-Systems (780) einem linken stereoskopischen Beobachtungsstrahlengang (706) wenigstens teilweise überlagert ist und mit diesem das Mikroskop-Hauptobjektiv (701) des Operationsmikroskops (700) durchsetzt.

15. Operationsmikroskop nach Anspruch 14, **dadurch gekennzeichnet, dass** das erste OCT-System (720) einen OCT-Abtastlichtstrahl (723) der Wellenlänge $\lambda_1$ = 1300nm bereitstellt und das zweite OCT-System (780) einen OCT-Abtastlichtstrahl (783) der Wellenlänge $\lambda_2$ = 800nm bereitstellt.

## Claims

1.  Operating microscope (100, 600, 700), comprising

    - an observation beam path (106, 203, 606, 705, 706); and
    - a microscope main objective system (101, 601, 701), through which the observation beam path (106, 203, 606, 705, 706) passes; and
    - an OCT system (120, 620, 720, 780) for examining an object region (108, 608, 708), wherein the OCT system (120, 620, 720, 780) has an OCT scanning beam path (123, 623, 723, 783) that is routed through the microscope main objective system (101, 601)

    **characterized in that**

    - a coupling-in element (150, 650, 750) is provided in the observation beam path (106) in order to couple the OCT scanning beam path (123, 623, 723, 783) into the observation beam path (106, 606, 705, 706) and route said OCT scanning beam path to the object region (108, 608, 708) through the microscope main objective system (101, 601, 701),
    - the coupling-in element is embodied as a splitter mirror, which is arranged in the observation beam path (106) and is substantially transparent to the spectral range of observation light that is visible to a human,
    - the splitter mirror reflects the OCT scanning beam path and superposes the latter onto the observation beam path (106, 606, 705, 706).

2.  Operating microscope according to Claim 1, **characterized in that** the coupling-in element is embodied as a plane mirror or a splitter cube.

3.  Operating microscope according to Claim 1 or 2, **characterized in that** one observation beam path (201, 202, 703, 704) is provided for the main observation and one observation beam path (106, 203, 606, 705, 706) is provided for co-observation, with both beam paths passing through the microscope main objective system (101, 601, 701), wherein the coupling-in element (150, 650, 750) is arranged in the observation beam path (106, 203, 606, 705, 706) for co-observation.

4.  Operating microscope according to Claim 3, **characterized in that** an optical assembly (109, 609) is arranged in the observation beam path (106, 606) for co-observation in order to transform a parallel observation beam path into an intermediate image (110, 610).

5.  Operating microscope according to Claim 4, **characterized in that** the coupling-in element (150) in the observation beam path (106) for co-observation is arranged between the optical assembly (109) and the microscope main objective system (101).

6.  Operating microscope according to Claim 4, **characterized in that** the coupling-in element (650) in the observation beam path (606) for co-observation is arranged between the optical assembly (609) and the intermediate image (610).

7.  Operating microscope according to one of Claims 1 to 6, **characterized in that** the OCT system (120, 620) comprises a first scanning mirror (124, 624) for scanning the OCT scanning beam path, which first scanning mirror can be moved about at least a first rotational axis (303).

8.  Operating microscope according to Claim 7, **characterized in that** a second scanning mirror (124, 125, 624, 625) that can be moved about a second rotational axis (304) is provided, with the first rotational axis (303) and the second rotational axis (304) being laterally offset and at a right angle with respect to one another.

9.  Operating microscope according to one of Claims 1 to 8, **characterized in that** the OCT system (120, 620) comprises an optical waveguide (122, 622), which has a light emission section for the OCT scanning beam path, with means being provided for moving the light emission section of the optical waveguide.

10. Operating microscope according to one of Claims 1 to 9, **characterized in that** an adjustable optical element (630, 631) is provided in the OCT scanning beam path (623) for setting a geometric image of an emission end of an optical waveguide (622) in an OCT scanning plane (660).

11. Operating microscope according to Claim 10, **characterized in that** a drive unit (672, 673) is assigned to the adjustable optical element (630, 631).

12. Operating microscope according to one of Claims 1 to 11, **characterized in that** the OCT system (120, 620) is designed for the provision of a first OCT scanning light beam (623) at a first wavelength and for the provision of a second OCT scanning light beam (623) at a second wavelength that differs from the first wavelength.

13. Operating microscope according to one of Claims 1 to 12, **characterized in that** provision is made for a first OCT system (720) and a second OCT system (780) that provide OCT scanning light beams (723, 783) with different wavelengths.

14. Operating microscope according to Claim 13, **characterized in that** the OCT scanning light beam (723) of the first OCT system (720) is at least partly superposed on a right stereoscopic observation beam path (705) and, together with the latter, passes through the microscope main objective system (701) of the operating microscope (700) and the OCT scanning light beam (783) of the second OCT system (780) is at least partly superposed on a left stereoscopic observation beam path (706) and, together with the latter, passes through the microscope main objective system (701) of the operating microscope (700).

15. Operating microscope according to Claim 14, **characterized in that** the first OCT system (720) provides an OCT scanning light beam (723) with a wavelength $\lambda_1$ = 1300 nm and the second OCT system (780) provides an OCT scanning light beam (783) with a wavelength $\lambda_2$ = 800 nm.

## Revendications

1. Microscope d'opération (100, 600, 700) avec

   - un chemin des rayons d'observation (106, 203, 606, 705, 706) ; et
   - un objectif principal de microscope (101, 601, 701) traversé par le chemin des rayons d'observation (106, 203, 606, 705, 706) ; et
   - un système OCT (120, 620, 720, 780) pour l'examen d'un espace objet (108, 608, 708), le système OCT (120, 620, 720, 780) comportant un chemin de rayons OCT de scrutation (123, 623, 723, 783) guidé à travers l'objectif principal de microscope (101, 601),

   **caractérisé en ce que**

   - dans le chemin de rayons d'observation (106), on a prévu un élément de couplage d'entrée (150, 650, 750) pour coupler l'entrée du chemin de rayons OCT de scrutation (123, 623, 723, 783) dans le chemin des rayons d'observation (106, 606, 705, 706) et pour le guider à travers l'objectif principal de microscope (101, 601, 701) vers l'espace objet (108, 608, 708),
   - l'élément de couplage d'entrée est réalisé sous la forme d'un miroir diviseur disposé dans le chemin de rayons d'observation (106) et est essentiellement transparent pour le domaine spectral visible pour l'humain de la lumière d'observation,

   - le miroir diviseur réfléchit le chemin de rayons OCT de scrutation et le superpose au chemin des rayons d'observation (106, 606, 705, 706).

2. Microscope d'opération selon la revendication 1, **caractérisé en ce que** l'élément de couplage d'entrée est réalisé sous la forme d'un miroir plan ou d'un prisme diviseur.

3. Microscope d'opération selon la revendication 1 ou 2, **caractérisé en ce qu'**on a prévu un chemin de rayons d'observation (201, 202, 703, 704) pour l'observation principale et un chemin de rayons d'observation (106, 203, 606, 705, 706) pour l'observation simultanée, traversant tous deux l'objectif principal de microscope (101, 601, 701), l'élément de couplage d'entrée (150, 650, 750) étant disposé dans le chemin des rayons d'observation (106, 203, 606, 705, 706) pour l'observation simultanée.

4. Microscope d'opération selon la revendication 3, **caractérisé en ce qu'**un module optique (109, 609) est disposé dans le chemin de rayons d'observation (106, 606) pour l'observation simultanée, pour transférer un chemin de rayons d'observation parallèle dans une image intermédiaire (110, 610).

5. Microscope d'opération selon la revendication 4, **caractérisé en ce que** l'élément de couplage d'entrée (150) est disposé dans le chemin des rayons d'observation (106) pour l'observation simultanée entre le module optique (109) et l'objectif principal du microscope (101).

6. Microscope d'opération selon la revendication 4, **caractérisé en ce que** l'élément de couplage d'entrée (650) est disposé dans le chemin des rayons d'observation (606) pour l'observation simultanée entre le module optique (609) et l'image intermédiaire (610).

7. Microscope d'opération selon une des revendications 1 à 6, **caractérisé en ce que** le système OCT (120, 620) pour le balayage du chemin de rayons OCT de scrutation comporte un premier miroir de balayage (124, 624) pouvant se déplacer autour d'au moins un premier axe de rotation (303).

8. Microscope d'opération selon la revendication 7, **caractérisé en ce qu'**on a prévu un deuxième miroir de balayage (124, 125, 624, 625) pouvant se déplacer autour d'un deuxième axe de rotation (304), le premier axe de rotation (303) et le deuxième axe de rotation (304) étant latéralement décalés à angle droit l'un par rapport à l'autre.

9. Microscope d'opération selon une des revendications 1 à 8, **caractérisé en ce que** le système OCT (120, 620) comporte un conduit de lumière (122, 622) présentant une section de sortie de la lumière pour le chemin de rayons OCT de scrutation, des moyens étant prévus pour le déplacement de la section de sortie de la lumière du conduit de lumière.

10. Microscope d'opération selon une des revendications 1 à 9, **caractérisé en ce que** dans le chemin de rayons OCT de scrutation (623), on a prévu un élément optique réglable (630, 631) pour le réglage d'une représentation géométrique d'une extrémité de sortie d'un conduit de lumière (622) dans un plan de scrutation OCT (660).

11. Microscope d'opération selon la revendication 10, **caractérisé en ce qu'**une unité d'entraînement (672, 673) est affectée à l'élément optique réglable (630, 631).

12. Microscope d'opération selon une des revendications 1 à 11, **caractérisé en ce que** le système OCT (120, 620) est conçu avec une première longueur d'ondes pour la mise à disposition d'un premier rayon lumineux OCT de scrutation (623) et avec une deuxième longueur d'ondes différente de la première longueur d'ondes pour la mise à disposition d'un deuxième rayon lumineux OCT de scrutation (623).

13. Microscope d'opération selon une des revendications 1 à 12, **caractérisé en ce qu'**on a prévu un premier système OCT (720) et un deuxième système OCT (780) qui mettent à disposition des rayons lumineux OCT de scrutation (723, 783) de longueurs d'ondes différentes.

14. Microscope d'opération selon la revendication 13, **caractérisé en ce que** le rayon lumineux OCT de scrutation (723) du premier système OCT (720) est au moins partiellement superposé à un chemin de rayons d'observation stéréoscopique droit (705) et traverse avec celui-ci l'objectif principal du microscope (701) du microscope d'opération (700) et le rayon lumineux OCT de scrutation (783) du deuxième système OCT (780) est au moins partiellement superposé à un chemin de rayons d'observation stéréoscopique gauche (706) et traverse avec celui-ci l'objectif principal du microscope (701) du microscope d'opération (700).

15. Microscope d'opération selon la revendication 14, **caractérisé en ce que** le premier système OCT (720) met à disposition un rayon lumineux OCT de scrutation (723) de longueur d'ondes $\lambda_1 = 1300$ nm et le deuxième système OCT (780) met à disposition un rayon lumineux OCT de scrutation (783) de longueur d'ondes $\lambda_2 = 800$ nm

**FIG.1**

## FIG.2

## FIG.3

# FIG.4

# FIG.5

**FIG.6**

**FIG.7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102004049368 A1
- EP 0815801 B1

- US 5321501 A
- WO 200610544 A1